# EUROPEAN PATENT SPECIFICATION

(11) **EP 2 295 040 B1**
(45) Date of publication and mention of the grant of the patent: **12.10.2011**
(21) Application number: 10175596.5
(22) Date of filing: 07.09.2010
(51) Int. Cl.: A61K 9/16, A61K 9/20, A61K 31/428

(54) **Pharmaceutical compositions of pramipexole**
Pharmazeutische Zusammensetzungen von Pramipexol
Compositions pharmaceutiques de pramipexole

(30) Priority: 11.09.2009 TR 200906997
(43) Date of publication of application: 16.03.2011
(73) Proprietor: Sanovel Ilac Sanayi ve Ticaret A.S., 34398 Istanbul (TR)
(72) Inventor: Toksöz, Ahmet, 34398 Istanbul (TR); Cifter, Ümit, 34398 Istanbul (TR); Türkyilmaz, Ali, 34398 Istanbul (TR); Yelken, Gülay, 34398 Istanbul (TR); Turp, Hasan Ali, 34398 Istanbul (TR); Öner, Levent, 06610 Ankara (TR)
(74) Representative: Sevinç, Erkan

(56) References cited:
- WO-A1-2008/129043
- WO-A2-2007/090881
- WO-A2-2008/023027
- WO-A2-2008/122638

## Description

### Technical Aspect

This invention is related to a novel pharmaceutical composition of pramipexole or a pharmaceutically acceptable salt, solvate and hydrate thereof, comprising sodium starch glycolate and dibasic calcium phosphate dihydrate with at least one pharmaceutically acceptable excipient, for oral administration, for their preparation and use thereof.

### Background of the Invention

Pramipexole is a nonergot dopamine agonist. The chemical name of pramipexole is (S)-2-amino-4,5,6,7-tetrahydro-6-(propylamino)benzothiazole and its chemical structure is shown in the Formula 1.

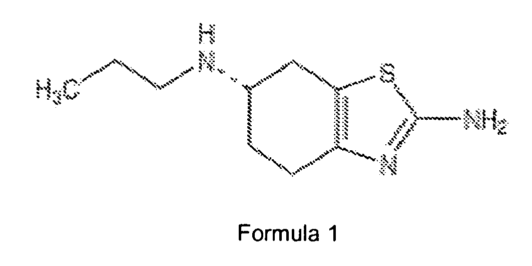

Pramipexole immediate release tablets are marketed under the name of Mirapex^{®} and are indicated to be taken 3 times a day for oral administration and contain 0.125 mg, 0.25 mg, 0.5 mg, 1 mg, or 1.5 mg of pramipexole dihydrochloride monohydrate as active ingredient.

Pramipexole and processes for preparing it are described in EP0186087B1 and it is known primarily for the treatment of schizophrenia and Parkinson's disease.

In prior art, there are many patents of immediate relase formulations of pramipexole dihydrochloride monohydrate and use therof such as EP0989850B1, EP1414446B1, EP1416930A1 and WO 2008/145252 A1. These Boehringer Ingelheim patents are related to the use of a compound having pramipexole dihydrochloride monohydrate as active ingredient and mannitol, maize starch, colloidal silicon dioxide, povidone, and magnesium stearate as excipients.

It is known that low dose pharmaceutical compositions have the problem of obtaining adequate content uniformity. Because it is difficult to homogenize the low active ingredient in final dosage form and some other difficulties may occur compressing them and this may cause inadequate content uniformity of the final dosage forms. Various processes can be used to get over this problem but in prior art there is no mention of content uniformity of the low dosage forms of pramipexole compositions.

In these two PCT applications WO 2008/023027 A2 and WO 2008/122638 A2, Boehringer Ingelheim Pharma Gmbh, a process for preparing tablets of pramipexole dihydrochloride is disclosed wherein the tablets exhibit high storage stability properties. However, there is no mention of content uniformity of the dosage forms of pramipexole dihydrochloride in these two patent applications.

WO 2008/129043 describes an extended release tablet formulation comprising pramipexole in the form of pellets which are coated with a sustained release layer.

It is also known in the pharmaceutical field that, when formulating a composition with low dose pharmaceutical active ingredients for administration to those in need of therapy, the challenge is to ensure an even distribution of the pharmaceutically active ingredients throughout the pharmaceutical excipients to ensure a proper dosage and homogeneity. Therefore, it would be desirable to provide improved processes for preparing solid oral dosage forms that have an adequate content uniformity and that disperse well upon oral administration.

As shown above many different approaches how to improve high storage stability of pramipexole or a pharmaceutically acceptable salt, solvate and hydrate thereof, have been disclosed in the prior art. Therefore, there is need in the art for pharmaceutical compositions of pramipexole or a pharmaceutically acceptable salt, solvate and hydrate thereof for oral administration, which has an adequate content uniformity causing a good dispersion upon oral administration and high bioavailability with improved processes and stability for their preparation and use thereof.

### Description of the Invention

The present invention provides a novel pharmaceutical composition of pramipexole or a pharmaceutically acceptable salt, solvate and hydrate thereof, comprising sodium starch glycolate and dibasic calcium phosphate dihydrate with at least one pharmaceutically acceptable excipient which overcomes the above described problems in prior art and have additive advantages over them.

The main object of the present invention is to obtain adequate content uniformity of low dosage forms of pramipexole or a pharmaceutically acceptable salt, solvate and hydrate thereof with using adequate excipients and improved processes.

A further object is to provide improved processes of pharmaceutical compositions of pramipexole or a pharmaceutically acceptable salt, solvate and hydrate thereof.

Another object of this invention is to provide an improved stability with high bioavailability of pharmaceutical compositions of pramipexole or a pharmaceutically acceptable salt, solvate and hydrate thereof.

In prior art, pharmaceutical compositions of pramipexole mostly have corn starch and mannitol with other common known excipients. However unmodified starch such as corn starch has poor flow characteristics and tends to increase tablet friability and capping if used in high concentrations.

We have found that, solid oral dosage forms such as tablets, when prepared with sodium starch glycolate have good storage properties. Additionally, the improved flow and lubricity characteristics of sodium starch glycolate can impart further benefit to the formulation in a very cost-effective manner.

Surprisingly it is found that when sodium starch glycolate and dibasic calcium phosphate dihydrate is used with pramipexole or a pharmaceutically acceptable salt, solvate and hydrate thereof, the pharmaceutical compositions of this invention has better storage stability and better compressibility with less friability. Thus, an adequate content uniformity is achieved in the final dosage forms.

In this pharmaceutical composition of the present invention, it is objected to use sodium starch glycolate with dibasic calcium phosphate dihydrate within a specific weight ratio to overcome the technical problems which are shown above. The weight ratio of sodium starch glycolate to dibasic calcium phosphate dihydrate is 1:100 to 10:1 (w/w), preferably the ratio is 1:50 to 5:1 (w/w), more preferably the ratio is 1:10 to 1:5 (w/w).

Another object of this present invention is a pharmaceutical composition of pramipexole or a pharmaceutically acceptable salt, solvate and hydrate thereof further comprising microcrystalline cellulose.

According to another preferred embodiment of the present invention, a synergistic effect is observed over the distribution of the low doses of pramipexole dihydrochloride monohydrate throughout other pharmaceutical excipients when dibasic calcium phosphate dihydrate and microcrystalline cellulose is used in a specific weight ratio wherein the weight ratio of dibasic calcium phosphate dihydrate to microcrystalline cellulose is 1:10 to 10:1 (w/w), preferably it is 1:3 (w/w). Thus, a proper dosage and homogeneity is ensured and the final dosage forms have high and adequate content uniformity.

In a preffered embodiment, the pharmaceutical composition of pramipexole or a pharmaceutically acceptable salt is in the form of dihydrochloride monohydrate salt.

In one aspect, the pharmaceutical composition of pramipexole dihydrochloride monohydrate is present in an amount of 0.01 to 5.0% by weight of total composition; preferably it is 0.1 to 2.0% by weight of total composition.

The pharmaceutical compositions of pramipexole dihydrochloride monohydrate comprise at least one pharmaceutically acceptable excipient. Suitable pharmaceutically acceptable excipients comprise but are not limited to disintegrants, fillers, diluents, binders, adhesives, glidants and lubricants.

Suitable disintegrants may comprise but not limited to sodium starch glycolate, croscarmellose sodium, crospovidone, algins, gums, surfactants and the like and mixtures thereof. The most preffered disintegrant is sodium starch glycolate.

Suitable fillers and/or diluents may comprise but not limited to dibasic calcium phosphate dihydrate, polysaccharides, primarily microcrystalline cellulose, lactose, mannitol, sugars, sorbitol, sucrose, inorganic salts, primarily calcium salts and the like and mixtures thereof. The most preffered fillers and/or diluents are dibasic calcium phosphate dihydrate and microcrystalline cellulose.

Suitable binders and/or adhesives may comprise but not limited to polyvinylpyrrolidone (povidone), gelatin, sugars, glucose, natural gums, synthetic celluloses, polymethycrylate, cellulose derivatives such as hydroxypropyl methyl cellulose, hydroxypropyl cellulose, carboxy methyl cellulose, methyl cellulose and the like and mixtures thereof. The most preffered binder and/or adhesive is polyvinylpyrrolidone.

Suitable glidants may comprise but not limited to colloidal silicon dioxide, talc, aluminium silicate and the like and mixtures thereof. The most preffered glidant is colloidal silicon dioxide.

Suitable lubricants may comprise but not limited to magnesium stearate, sodium stearyl fumarate, polyethylene glycol, stearic acid, metal stearates, talc, boric acid, sodium chloride benzoate and acetate, sodium or magnesium lauryl sulfate and the like and mixtures thereof. The most preffered lubricant is magnesium stearate.

In one aspect, the pharmaceutical composition of pramipexole dihydrochloride monohydrate comprises,
a. 0.01 - 5.0 % of pramipexole dihydrochloride monohydrate
b. 5.0 - 90 % of dibasic calcium phosphate dihydrate
c. 0.1 - 10 % of sodium starch glycolate
d. 5.0 - 90 % of microcrystalline cellulose
e. 0.1 - 25 % of polyvinylpyrolidone
f. 0.01 - 2.0 % of colloidal silicon dioxide
g. 0.1 - 5.0 % of magnesium stearate

In particular, the dosage form of the present invention is a solid dosage form such as tablets, capsules, powders, sachets, etc. The preferred dosage form is tablet.

In one of the preffered embodiments of this invention, the final dosage form of the pharmaceutical composition of pramipexole dihydrochloride monohydrate has a content uniformity of less than 2.0 % RSD (Relative Standard Deviation), preferably it has a content uniformity of less than 1.0 % RSD.

In one of the preffered embodiments of this invention the pharmaceutical composition of pramipexole dihydrochloride monohydrate may have a long-term shelf-life of 24 months or more at ambient temperature, in its original packaging.

The pharmaceutical compositions of this invention are suitable for the treatment of Parkinsonism or parkionson's disease and complications or disorders associated therewith, schizophrenia and restless leg syndrome.

In one embodiment, the pharmaceutical compositions according to the present invention may further comprise one or more other active ingredients such as L-dopa.

The pharmaceutical compositions of the present invention may be prepared by conventional technology well known to those skilled in the art such as wet granulation, dry granulation and direct compression and the like. The manufacturing process is preferably performed by wet granulation.

The preffered wet granulation process of the present invention for preparing the pharmaceutical composition of pramipexole dihydrochloride monohydrate comprises the following steps;
a. dissolving the pramipexole dihydrochloride monohydrate and polyvinylpyrrollidone in water and/or alcohol to form an aqueous pramipexole dihydrochloride monohydrate solution,
b. while the solution is mixing, dibasic calcium phosphate dihydrate, microcrystalline cellulose and half part of sodium starch glycolate are added and then blended in a high-shear granulator to form granules,
c. sieving and drying the wet granules and milling the dried granules,
d. adding colloidal silicon dioxide and the rest of the sodium starch glycolate and mixing them,
e. adding magnesium stearate to this mixture and blending them until obtaining a homogenous powder mixture,
f. compressing the blended mixture to form tablets or filling the powder mixture into capsules.

Second preferred wet granulation process of the present invention for preparing the pharmaceutical composition of pramipexole dihydrochloride monohydrate comprises the following steps;
a. dissolving the pramipexole dihydrochloride monohydrate and polyvinylpyrrollidone in water and/or alcohol to form an aqueous pramipexole dihydrochloride monohydrate solution,
b. mixing dibasic calcium phosphate dihydrate, microcrystalline cellulose and half part of sodium starch glycolate in a high-shear granulator,
c. spraying the pramipexole dihydrochloride monohydrate solution to the blended powder mixture of dibasic calcium phosphate dihydrate, sodium starch glycolate and microcrystalline cellulose,
d. sieving and drying the wet granules and milling the dried granules,
e. adding colloidal silicon dioxide and the rest of the sodium starch glycoloate and mixing them,
f. adding magnesium stearate to this mixture and blending them until obtaining a homogenous powder mixture,
g. compressing the blended mixture to form tablets or filling the powder mixture into capsules.

The preferred direct compression process of the present invention for preparing the pharmaceutical composition of pramipexole dihydrochloride monohydrate comprises the following steps;
a. after sieving colloidal silicon dioxide and dibasic calcium phosphate anhydrous, they are mixed wherein the mixing time is preferably 5 min.
b. adding pramipexole dihydrochloride monohydrate to this powder mixture and mixing them, wherein the mixing time is preferably 15 min.
c. after sieving sodium starch glycolate, microcrystalline cellulose and copovidone, they are added to the powder mixture and mixed for about 5 min.
d. after sieving magnesium stearate, it is added to the powder mixture and mixed for about 3 min.
e. compressing the final powder mixture to form tablets or filling the powder mixture into capsules.

The pharmaceutical composition according to the present invention further comprise one or more other active ingredients, such as L-dopa; these ingredients can be added in any step of the manufacturing process as described above.

One of the preffered embodiments of this invention is using high shear granulator during wet granulation process instead of fluid bed dryer to get over some technical problems which are known in the pharmaceutical field such as described below;
a. to obtain a good flowability and compressibility of the final blended powder mixture,
b. to obtain hard granules at the end of the process which lessens the dustiness, and this provides less friable granules,
c. narrowing the particule size distribution of the powder mixtures,
d. to ensure an even distribution of the pharmaceutically active ingredient throughout the pharmaceutical excipients to ensure a proper dosage and homogeneity and this has an additive effect over content uniformity,
e. using less binder solution and this makes the timing of the process shorter,
f. to improve dissolution characteristics of the final dosage forms.

In one aspect, sieving step of the wet granulation processes is an important criteria for the content uniformity of the final dosage forms. According to this aspect, granules are sieved from a sieve which has a size of 0.50mm, preferably 0.25 mm.

The processes of the present invention is particularly useful for the preparation of dosage forms having an adequate content uniformity wherein the final dosage form has a content uniformity of less than 2.0 % RSD (Relative Standard Deviation), preferably less than 1.0 % RSD. Herein, content uniformity test is determined by calculating RSD (Relative Standard Deviation) value of 10 tablets.

The pharmaceutical compositions of this present invention (Ex 2), were tested for the content uniformity against the pramipexole composition including mannitol and corn starch instead of sodium starch glycolate, dibasic calcium phosphate dihydrate and microcrystalline cellulose. Ex 2 is one of the pharmaceutical compositions of pramipexole dihydrochloride monohydrate including sodium starch glycolate, dibasic calcium phosphate dihydrate, microcrystalline cellulose, polyvinylpyrrolidone (povidone), colloidal silicon dioxide and magnesium stearate (Table 1).

**Table 1 : Content Uniformity test results (%)**

| ***Sample No*** | ***Ex 2-1*** | ***Ex 2-2*** | ***Referance Product*** |
|---|---|---|---|
| **1** | 99,42 | 100,93 | 96,31 |
| **2** | 97,93 | 102,5 | 97,48 |
| **3** | 99,28 | 102,08 | 95,54 |
| **4** | 99,12 | 101,49 | 97,03 |
| **5** | 97,78 | 101,41 | 98,03 |
| **6** | 97,66 | 101,63 | 96,19 |
| **7** | 97,75 | 101,8 | 92,06 |
| **8** | 97,79 | 102,7 | 96,84 |
| **9** | 98,65 | 102,05 | 98,64 |
| **10** | 98,39 | 100,47 | 98,2 |
| **mean** | 98,38 | 101,71 | 96,63 |
| **SDEV** | 0,69 | 0,68 | 1,88 |
| **% RSD** | **0,71** | **0,67** | **1,94** |
| **Min.** | 97,66 | 100,47 | 92,06 |
| **Max.** | 99,42 | 102,70 | 98,64 |

**Reference Product**: Pramipexole dihydrochloride monohydrate as active ingredient and Reference Product: Pramipexole dihydrochloride monohydrate as active ingredient and mannitol, corn starch, colloidal silicon dioxide, povidone, magnesium stearate as inactive ingredients

These formulations are manufactured according to the wet granulation processes described above. Ex 2-1 is manufactured by the first wet granulation process described above and Ex 2-2 is manufactured by the second wet granulation process described above. During these manufacturing processes 0.25 mm size sieve is used.

This invention is further defined by reference to the following examples. Although the examples are not intended to limit the scope of the present invention, they should be considered in the light of the description detailed above. It will be apparent to those skilled in the art that many modifications, both to materials and methods, may be practiced without departing from the scope of the invention.

### Example 1 :

| **Ingredients** | **Amount mg** |
|---|---|
| Pramipexole dihydrochloride monohydrate | 0.125 |
| Dibasic calcium phosphate dihydrate | 19.686 |
| Sodium starch glycolate | 3.400 |
| Microcrystalline cellulose (Avicel pH-301) | 59.154 |
| Polyvinylpyrollidone (Povidone K-30) | 1.020 |
| Colloidal silicon dioxide | 0.340 |
| Magnesium stearate | 1.275 |
| Total tablet weight | 85.00 |

### Example 2 :

| **Ingredients** | **Amount mg** |
|---|---|
| Pramipexole dihydrochloride monohydrate | 0.25 |
| Dibasic calcium phosphate dihydrate | 24.32 |
| Sodium starch glycolate | 4.20 |
| Microcrystalline cellulose (Avicel pH-301) | 72.97 |
| Polyvinylpyrollidone (Povidone K-30) | 1.26 |
| Colloidal silicon dioxide | 0.42 |
| Magnesium stearate | 1.58 |
| Total tablet weight | 105.00 |

### Example 3:

| **Ingredients** | **Amount mg** |
|---|---|
| Pramipexole dihydrochloride monohydrate | 0.50 |
| Dibasic calcium phosphate dihydrate | 48.64 |
| Sodium starch glycolate | 8.40 |
| Microcrystalline cellulose (Avicel pH-301) | 145.94 |
| Polyvinylpyrollidone (Povidone K-30) | 2.52 |
| Colloidal silicon dioxide | 0.84 |
| Magnesium stearate | 3.16 |
| Total tablet weight | 210.00 |

### Example 4:

| **Ingredients** | **Amount mg** |
|---|---|
| Pramipexole dihydrochloride monohydrate | 1.00 |
| Dibasic calcium phosphate dihydrate | 48.64 |
| Sodium starch glycolate | 8.40 |
| Microcrystalline cellulose (Avicel pH-301) | 145.45 |
| Polyvinylpyrollidone (Povidone K-30) | 2.52 |
| Colloidal silicon dioxide | 0.84 |
| Magnesium stearate | 3.15 |
| Total tablet weight | 210.00 |

### Example 5:

| **Ingredients** | **Amount mg** |
|---|---|
| Pramipexole dihydrochloride monohydrate | 1.25 |
| Dibasic calcium phosphate dihydrate | 64.85 |
| Sodium starch glycolate | 11.20 |
| Microcrystalline cellulose (Avicel pH-301) | 194.02 |
| Polyvinylpyrollidone (Povidone K-30) | 3.36 |
| Colloidal silicon dioxide | 1.12 |
| Magnesium stearate | 4.20 |
| Total tablet weight | 280.00 |

### Example 6:

| **Ingredients** | **Amount mg** |
|---|---|
| Pramipexole dihydrochloride monohydrate | 1.50 |
| Dibasic calcium phosphate dihydrate | 83.38 |
| Sodium starch glycolate | 14.40 |
| Microcrystalline cellulose (Avicel pH-301) | 249.56 |
| Polyvinylpyrollidone (Povidone K-30) | 4.32 |
| Colloidal silicon dioxide | 1.44 |
| Magnesium stearate | 5.40 |
| Total tablet weight | 360.00 |

The formulations of these examples are manufactured according to the processes described detailed above in the description and tablet formulations may further comprise a coating.

## Claims

1. A pharmaceutical composition of pramipexole or a pharmaceutically acceptable salt, solvate and hydrate thereof, comprising sodium starch glycolate and dibasic calcium phosphate dihydrate with at least one pharmaceutically acceptable excipient.

2. The pharmaceutical composition of pramipexole or a pharmaceutically acceptable salt, solvate and hydrate thereof according to the claim 1, wherein the weight ratio of sodium starch glycolate to dibasic calcium phosphate dihydrate is 1:100 to 10:1 (w/w).

3. The pharmaceutical composition of pramipexole or a pharmaceutically acceptable salt, solvate and hydrate thereof according to the claim 2, wherein the weight ratio of sodium starch glycolate to dibasic calcium phosphate dihydrate is 1:50 to 5:1 (w/w).

4. The pharmaceutical composition of pramipexole or a pharmaceutically acceptable salt, solvate and hydrate thereof according to the claim 3, wherein the weight ratio of sodium starch glycolate to dibasic calcium phosphate dihydrate is 1:10 to 1:5 (w/w).

5. The pharmaceutical composition of pramipexole or a pharmaceutically acceptable salt, solvate and hydrate thereof according to any preceding claims, further comprising microcrystalline cellulose.

6. The pharmaceutical composition of pramipexole or a pharmaceutically acceptable salt, solvate and hydrate according to the claims 1 to 5, wherein the weight ratio of dibasic calcium phosphate dihydrate to microcrystalline cellulose is 1:10 to 10:1 (w/w).

7. The pharmaceutical composition of pramipexole or a pharmaceutically acceptable salt, solvate and hydrate according to the claim 6, wherein the weight ratio of dibasic calcium phosphate dihydrate to microcrystalline cellulose is 1:3 (w/w).

8. The pharmaceutical composition of pramipexole or a pharmaceutically acceptable salt, solvate and hydrate thereof according to any preceding claims, wherein pramipexole is in the form of dihydrochloride monohydrate salt.

9. The pharmaceutical composition of pramipexole dihydrochloride monohydrate according to claims 1 to 8, wherein pramipexole dihydrochloride monohydrate is present in an amount of 0.01 to 5.0% by weight of total composition; preferably it is 0.1 to 2.0% by weight of total composition.

10. The pharmaceutical composition of pramipexole dihydrochloride monohydrate according to the claims 1 to 9, wherein at least one pharmaceutically acceptable excipient is selected from the group comprising disintegrants, fillers, diluents, binders, adhesives, glidants and lubricants.

11. The pharmaceutical composition of pramipexole dihydrochloride monohydrate according to the claim 10, wherein the disintegrant is selected from the group comprising sodium starch glycolate, croscarmellose sodium, crospovidone, algins, gums, surfactants and the like and mixtures thereof; preferably the disintegrant is sodium starch glycolate.

12. The pharmaceutical composition of pramipexole dihydrochloride monohydrate according to the claim 10, wherein the filler and/or diluent is selected from the group comprising dibasic calcium phosphate dihydrate, polysaccharides, primarily microcrystalline cellulose, lactose, mannitol, sugars, sorbitol, sucrose, inorganic salts, primarily calcium salts and the like and mixtures thereof; preferably the fillers and/or diluents are microcrystalline cellulose and dibasic calcium phosphate dihydrate.

13. The pharmaceutical composition of pramipexole dihydrochloride monohydrate according to the claim 10, wherein the binder and/or adhesive is selected from the group comprising polyvinylpyrrolidone, gelatin, sugars, glucose, natural gums, synthetic celluloses, polymethycrylate, cellulose derivatives such as hydroxypropyl methyl cellulose, hydroxypropyl cellulose, carboxy methyl cellulose, methyl cellulose and the like and mixtures thereof; preferably the binder and/or adhesive is polyvinylpyrrolidone.

14. The pharmaceutical composition of pramipexole dihydrochloride monohydrate according to the claim 10, wherein the glidant is selected from the group comprising colloidal silicon dioxide, talc, aluminium silicate and the like and mixtures thereof; preferably the glidant is colloidal silicon dioxide.

15. The pharmaceutical composition of pramipexole dihydrochloride monohydrate according to the claim 10, wherein the lubricant is selected from the group comprising magnesium stearate, sodium stearyl fumarate, polyethylene glycol, stearic acid, metal stearates, talc, boric acid, sodium chloride benzoate and acetate, sodium or magnesium lauryl sulfate and the like and mixtures thereof; preferably the lubricant is magnesium stearate.

16. The pharmaceutical composition of pramipexole dihydrochloride monohydrate according to claims 1 to 15, comprising;
a. 0.01 - 5.0 % of pramipexole dihydrochloride monohydrate
b. 5.0 - 90 % of dibasic calcium phosphate dihydrate
c. 0.1 - 10 % of sodium starch glycolate
d. 5.0 - 90 % of microcrystalline cellulose
e. 0.1 - 25 % of polyvinylpyrolidone
f. 0.01 - 2.0 % of colloidal silicon dioxide
g. 0.1 - 5.0 % of magnesium stearate

17. The pharmaceutical composition of pramipexole dihydrochloride monohydrate according to any preceding claims, wherein the pharmaceutical composition is in the form of tablets, capsules, powders, sachets; preferably the final dosage form is in the form of a tablet.

18. The pharmaceutical composition of pramipexole dihydrochloride monohydrate according to any preceding claim, wherein the final dosage form has a content uniformity of less than 2.0% RSD (Relative Standard Deviation).

19. The pharmaceutical composition of pramipexole dihydrochloride monohydrate according to claim 18, wherein the final dosage form has a content uniformity of less than 1.0 % RSD (Relative Standard Deviation).

20. The pharmaceutical composition of pramipexole dihydrochloride monohydrate, according to any preceding claims, wherein the pharmaceutical composition has a long term shelf-life of 24 months or more at ambient temperature, in its original packaging.

21. A process for preparing the pharmaceutical composition of pramipexole dihydrochloride monohydrate according to any preceding claims, comprising the following steps;
a. dissolving pramipexole dihydrochloride monohydrate and polivinylpyrollidone in water and/or alcohol to form an aqueous pramipexole dihydrochloride monohydrate solution,
b. while the solution is mixing, dibasic calcium phosphate dhydrate, microcrystalline cellulose and half part of sodium starch glycoloate is added and blended in a high-shear granulator to form granules,
c. sieving and drying the wet granules and milling the dried granules,
d. adding colloidal silicon dioxide and the rest of the sodium starch glycoloate and mixing them,
e. adding magnesium stearate to this mixture and blending them until obtaining a homogenous powder mixture,
f. compressing the blended mixture to form tablets or filling the powder mixture into capsules.

22. A process for preparing the pharmaceutical composition of pramipexole dihydrochloride monohydrate according to any of claims 1-20 comprising the following steps;
a. Dissolving pramipexole dihydrochloride monohydrate and polivinylpyrollidone in water and/or alcohol to form an aqueous pramipexole dihydrochloride monohydrate solution,
b. mixing dibasic calcium phosphate dihydrate, microcrystalline cellulose and half part of sodium starch glycoloate in a high-shear granulator,
c. spraying the pramipexole dihydrochloride monohydrate solution to the blended powder mixture of dibasic calcium phosphate dihydrate, sodium starch glycoloate and microcrystalline cellulose,
d. sieving and drying the wet granules and milling the dried granules,
e. Adding colloidal silicon dioxide and the rest of the sodium starch glycoloate and mixing them,
f. Adding magnesium stearate to this mixture and blending them until obtaining a homogenous powder mixture,
g. compressing the blended mixture to form tablets or filling the powder mixture into capsules.

23. The process according to any preceding claim, wherein the sieve has a size of 0.50mm, preferably 0.25 mm.

24. Use of the pharmaceutical composition of pramipexole dihydrochloride monohydrate according to any preceding claims for preparing a pharmaceutical compositon for the treatment of Parkinsonism or parkinson's disease and complications or disorders associated therewith, schizophrenia and restless leg syndrome.

## Patentansprüche

1. Pharmazeutische Pramipexol-Zusammensetzung oder pharmazeutisch unbedenkliches Salz, Solvat und Hydrat derselben, umfassend Carboxymethylstärke-Natrium und zweibasiges Calciumphosphat-Dihydrat mit mindestens einem pharmazeutisch unbedenklich Hilfsstoff.

2. Pharmazeutische Pramipexol-Zusammensetzung oder pharmazeutisch unbedenkliches Salz, Solvat und Hydrat derselben nach Anspruch 1, wobei das Gewichtsverhältnis des Carboxymethylstärke-Natriums zum zweibasigen Calciumphosphat-Dihydrat 1:100 bis 10:1 (w/w) beträgt.

3. Pharmazeutische Pramipexol-Zusammensetzung oder pharmazeutisch unbedenkliches Salz, Solvat und Hydrat derselben nach Anspruch 2, wobei das Gewichtsverhältnis des Carboxymethylstärke-Natriums zum zweibasigen Calciumphosphat-Dihydrat 1:50 bis 5:1 (w/w) beträgt.

4. Pharmazeutische Pramipexol-Zusammensetzung oder pharmazeutisch unbedenkliches Salz, Solvat und Hydrat derselben nach Anspruch 3, wobei das Gewichtsverhältnis des Carboxymethylstärke-Natriums zum zweibasigen Calciumphosphat-Dihydrat 1:10 bis 1:5 (w/w) beträgt.

5. Pharmazeutische Pramipexol-Zusammensetzung oder pharmazeutisch unbedenkliches Salz, Solvat und Hydrat derselben nach einem beliebigen der vorhergehenden Ansprüche, weiterhin mikrokristalline Cellulose umfassend.

6. Pharmazeutische Pramipexol-Zusammensetzung oder pharmazeutisch unbedenkliches Salz, Solvat und Hydrat nach den Ansprüchen 1 bis 5, wobei das Gewichtsverhältnis des zweibasigen Calciumphosphat-Dihydrats zur mikrokristallinen Cellulose 1:10 bis 10:1 (w/w) beträgt.

7. Pharmazeutische Pramipexol-Zusammensetzung oder pharmazeutisch unbedenkliches Salz, Solvat und Hydrat nach Anspruch 6, wobei das Gewichtsverhältnis des zweibasigen Calciumphosphat-Dihydrats zur mikrokristallinen Cellulose 1:3 (w/w) beträgt.

8. Pharmazeutische Pramipexol-Zusammensetzung oder pharmazeutisch unbedenkliches Salz, Solvat und Hydrat derselben nach einem beliebigen der vorhergehenden Ansprüche, wobei Pramipexol in Form des Dihydrochlorid-Monohydrat-Salzes vorliegt.

9. Pharmazeutische Zusammensetzung von Pramipexol-Dihydrochlorid-Monohydrat nach den Ansprüche 1 bis 8, wobei das Pramipexol-Dihydrochlorid-Monohydrat in einer Menge von 0,01 bis 5,0 Gewichts% der Zusammensetzung insgesamt vorliegt; vorzugsweise macht es 0,1 bis 2,0 Gewichts% der Zusammensetzung insgesamt aus.

10. Pharmazeutische Zusammensetzung von Pramipexol-Dihydrochlorid-Monohydrat nach den Ansprüchen 1 bis 9, wobei mindestens ein pharmazeutisch unbedenklicher Hilfsstoff aus der Gruppe ausgewählt ist, die Sprengmittel, Füllstoffe, Verdünnungsmittel, Bindemittel, Klebstoffe, Fließregulierungsmittel und Gleitmittel umfasst.

11. Pharmazeutische Zusammensetzung von Pramipexol-Dihydrochlorid-Monohydrat nach Anspruch 10, wobei das Sprengmittel aus der Gruppe ausgewählt ist, die Carboxymethylstärke-Natrium, Croscarmellose-Natrium, Crospovidon, Alginate, Gummi, Tenside und Ähnliches sowie deren Mischungen umfasst, vorzugsweise handelt es sich bei dem Sprengmittel um Carboxymethylstärke-Natrium.

12. Pharmazeutische Zusammensetzung von Pramipexol-Dihydrochlorid-Monohydrat nach Anspruch 10, wobei der Füllstoff und/oder das Verdünnungsmittel aus der Gruppe ausgewählt ist, die zweibasiges Calciumphosphat-Dihydrat, Polysaccharide, in erster Linie mikrokristalline Cellulose, Lactose, Mannit, Zucker, Sorbit, Saccharose, anorganische Salze, in erster Linie Calciumsalze und Ähnliches sowie deren Mischungen umfasst; vorzugsweise handelt es sich bei den Füllstoffen und/oder Verdünnungsmitteln um mikrokristalline Cellulose und zweibasiges Calciumphosphat-Dihydrat.

13. Pharmazeutische Zusammensetzung von Pramipexol-Dihydrochlorid-Monohydrat nach Anspruch 10, wobei das Bindemittel und/oder der Klebstoff aus der Gruppe ausgewählt ist, die Polyvinylpyrrolidon, Gelatine, Zucker, Glucose, Naturgummi, synthetische Cellulosen, Polymethycrylat, Cellulosederivate wie etwa Hydroxylpropylmethylcellulose, Hydroxylpropylcellulose, Carboxymethylcellulose, Methylcellulose und Ähnliches sowie deren Mischungen umfasst; vorzugsweise handelt es sich bei dem Bindemittel und/oder Klebstoff um Polyvinylpyrrolidon.

14. Pharmazeutische Zusammensetzung von Pramipexol-Dihydrochlorid-Monohydrat nach Anspruch 10, wobei das Fließregulierungsmittel aus der Gruppe ausgewählt ist, die kolloidales Siliciumdioxid, Talk, Aluminiumsilicat und Ähnliches sowie deren Mischungen umfasst; vorzugsweise handelt es sich bei dem Fließregulierungsmittel um kolloidales Siliciumdioxid.

15. Pharmazeutische Zusammensetzung von Pramipexol-Dihydrochlorid-Monohydrat nach Anspruch 10, wobei das Gleitmittel aus der Gruppe ausgewählt ist, die Magnesiumstearat, Natriumstearylfumarat, Polyethylenglykol, Stearinsäure, Metallstearate, Talk, Borsäure, Natriumchlorid, -benzoat und -acetat, Natrium- oder Magnesiumlaurylsulfat und Ähnliches sowie deren Mischungen umfasst; vorzugsweise handelt es sich bei dem Gleitmittel um Magnesiumstearat.

16. Pharmazeutische Zusammensetzung von Pramipexol-Dihydrochlorid-Monohydrat nach den Ansprüchen 1 bis 15, umfassend;
a) 0,01 bis 5,0 % an Pramipexol-Dihydrochlorid-Monohydrat
b) 5,0 bis 90 % an zweibasigem Calciumphosphat-Dihydrat
c) 0,1 bis 10 % an Carboxymethylstärke-Natrium
d) 5,0 % bis 90 % mikrokristalliner Cellulose;
e) 0,1 bis 25 % an Polyvinylpyrrolidon
f) 0,01 bis 2,0 an kolloidalem Siliciumdioxid
g) 0,1 bis 5,0 % an Magnesiumstearat

17. Pharmazeutische Zusammensetzung von Pramipexol-Dihydrochlorid-Monohydrat nach einem beliebigen der vorhergehenden Ansprüche, wobei die pharmazeutische Zusammensetzung in Form von Tabletten, Kapseln, Pulver, Dosierbeuteln vorliegt; vorzugsweise handelt es sich bei der Dosierform für den Endanwender um eine Tablette.

18. Pharmazeutische Zusammensetzung von Pramipexol-Dihydrochlorid-Monohydrat nach einem beliebigen der vorhergehenden Ansprüche, wobei die Dosierform für den Endanwender eine gehaltsbezogene Gleichförmigkeit mit einer RSD (relativen Standardabweichung) von weniger als 2,0 % hat.

19. Pharmazeutische Zusammensetzung von Pramipexol-Dihydrochlorid-Monohydrat nach Anspruch 18, wobei die Dosierform für den Endanwender eine gehaltsbezogene Gleichförmigkeit mit einer RSD (relativen Standardabweichung) von weniger als 1,0 % hat.

20. Pharmazeutische Zusammensetzung von Pramipexol-Dihydrochlorid-Monohydrat nach einem beliebigen der vorhergehenden Ansprüche, wobei die pharmazeutische Zusammensetzung bei Raumtemperatur, in ihrer Originalverpackung, eine langfristige Lagerfähigkeit von 24 Monaten hat.

21. Verfahren zur Herstellung der pharmazeutischen Zusammensetzung von Pramipexol-Dihydrochlorid-Monohydrat nach einem beliebigen der vorhergehenden Ansprüche, die folgenden Schritte umfassend;
a) Auflösen von Pramipexol-Dihydrochlorid-Monohydrat und Polyvinylpyrrolidon in Wasser und/oder Alkohol, um eine wässrige Lösung von Pramipexol-Dihydrochlorid-Monohydrat zu bilden,
b) während des Vermischens der Lösung wird zweibasiges Calciumphosphat-Dihydrat, mikrokristalline Cellulose und die Hälfte an Carboxymethylstärke-Natrium zugesetzt und in einer Granulatmaschine mit großer Scherkraft durchmischt, um Granulatkörner zu bilden,
c) Sieben und Trocknen der feuchten Granulatkörner und Zerkleinern der getrockneten Granulatkörner,
d) Hinzufügen kolloidalen Siliciumdioxids und des Restes an Carboxymethylstärke-Natrium, und Vermischen derselben,
e) Hinzufügen von Magnesiumstearat zu dieser Mischung und Durchmischen derselben, bis eine homogene Pulvermischung erhalten wird,
f) Komprimieren der gleichförmigen Mischung, um Tabletten zu bilden, oder Einfüllen der Pulvermischung in Kapseln.

22. Verfahren zur Herstellung der pharmazeutischen Zusammensetzung von Pramipexol-Dihydrochlorid-Monohydrat nach einem beliebigen der Ansprüche 1 bis 20, die folgenden Schritte umfassend;
a) Auflösen von Pramipexol-Dihydrochlorid-Monohydrat und Polyvinylpyrrolidon in Wasser und/oder Alkohol, um eine wässrige Lösung von Pramipexol-Dihydrochlorid-Monohydrat zu bilden,
b) Vermischen von zweibasigem Calciumphosphat-Dihydrat, mikrokristalliner Cellulose und der Hälfte an Carboxymethylstärke-Natrium in einer Granulatmaschine mit großer Scherkraft,
c) Einsprühen der Lösung von Pramipexol-Dihydrochlorid-Monohydrat in die durchmischte Pulvermischung von Calciumphosphat-Dihydrat, mikrokristalliner Cellulose und Carboxymethylstärke-Natrium,
d) Sieben und Trocknen der feuchten Granulatkörner und Zerkleinern der getrockneten Granulatkörner,
e) Hinzufügen kolloidalen Siliciumdioxids und des Restes an Carboxymethylstärke-Natrium, und Vermischen derselben,
f) Hinzufügen von Magnesiumstearat zu dieser Mischung und Durchmischen derselben, bis eine homogene Pulvermischung erhalten wird,
g) Komprimieren der gleichförmigen Mischung, um Tabletten zu bilden, oder Einfüllen der Pulvermischung in Kapseln.

23. Verfahren nach einem beliebigen vorhergehenden Anspruch, wobei das Sieb eine Größe von 0,50 mm, vorzugsweise von 0,25 mm, hat.

24. Verwendung der pharmazeutischen Zusammensetzung von Pramipexol-Dihydrochlorid-Monohydrat nach einem beliebigen der vorhergehenden Ansprüche zur Herstellung einer pharmazeutischen Zusammensetzung zur Behandlung von Parkinsonismus oder der Parkinson-Krankheit und den damit verbundenen Komplikationen und Störungen, von Schizophrenie und des Syndroms der unruhigen Beine.

## Revendications

1. Composition pharmaceutique de pramipexole ou d'un sel, solvate et hydrate de celui-ci pharmaceutiquement acceptables, comprenant du glycolate d'amidon sodique et du phosphate dicalcique dihydraté avec au moins un excipient pharmaceutiquement acceptable.

2. Composition pharmaceutique de pramipexole ou d'un sel, solvate et hydrate de celui-ci pharmaceutiquement acceptables selon la revendication 1, dans laquelle le rapport pondéral du glycolate d'amidon sodique au phosphate dicalcique dihydraté est de 1:100 à 10:1 (p/p).

3. Composition pharmaceutique de pramipexole ou d'un sel, solvate et hydrate de celui-ci pharmaceutiquement acceptables selon la revendication 2, dans laquelle le rapport pondéral du glycolate d'amidon sodique au phosphate dicalcique dihydraté est de 1:50 à 5:1 (p/p).

4. Composition pharmaceutique de pramipexole ou d'un sel, solvate et hydrate de celui-ci pharmaceutiquement acceptables selon la revendication 3, dans laquelle le rapport pondéral du glycolate d'amidon sodique au phosphate dicalcique dihydraté est de 1:10 à 1:5 (p/p).

5. Composition pharmaceutique de pramipexole ou d'un sel, solvate et hydrate de celui-ci pharmaceutiquement acceptables selon l'une quelconque des revendications précédentes, comprenant en outre de la cellulose microcristalline.

6. Composition pharmaceutique de pramipexole ou d'un sel, solvate et hydrate de celui-ci pharmaceutiquement acceptables selon les revendications 1 à 5, dans laquelle le rapport pondéral du phosphate dicalcique dihydraté à la cellulose microcristalline est de 1:10 à 10:1 (p/p).

7. Composition pharmaceutique de pramipexole ou d'un sel, solvate et hydrate pharmaceutiquement acceptables selon la revendication 6, dans laquelle le rapport pondéral du phosphate dicalcique dihydraté à la cellulose microcristalline est de 1:3 (p/p).

8. Composition pharmaceutique de pramipexole ou d'un sel, solvate et hydrate de celui-ci pharmaceutiquement acceptables selon l'une quelconque des revendications précédentes, dans laquelle le pramipexole est sous la forme de sel de dichlorhydrate monohydraté.

9. Composition pharmaceutique de dichlorhydrate de pramipexole monohydraté selon les revendications 1 à 8, dans laquelle le dichlorhydrate de pramipexole monohydraté est présent dans une quantité de 0,01 à 5,0% en poids de la composition totale ; de préférence, il fait 0,1 à 2,0% en poids de la composition totale.

10. Composition pharmaceutique de dichlorhydrate de pramipexole monohydraté selon les revendications 1 à 9, dans laquelle au moins un excipient pharmaceutiquement acceptable est sélectionné parmi le groupe comprenant les délitants, les charges, les diluants, les liants, les adhésifs, les glissants et les lubrifiants.

11. Composition pharmaceutique de dichlorhydrate de pramipexole monohydraté selon la revendication 10, dans laquelle le délitant est sélectionné parmi le groupe comprenant le glycolate d'amidon sodique, le sodium de croscarmellose, la crospovidone, les algines, les gommes, les agents de surface et équivalents et mélanges de ceux-ci ; de préférence, le délitant est du glycolate d'amidon sodique.

12. Composition pharmaceutique de dichlorhydrate de pramipexole monohydraté selon la revendication 10, dans laquelle la charge et/ou le diluant est sélectionné(e) parmi le groupe comprenant le phosphate dicalcique dihydraté, les polysaccharides, principalement la cellulose microcristalline, le lactose, le mannitol, les sucres, le sorbitol, le saccharose, les sels inorganiques, principalement les sels de calcium et équivalents et mélanges de ceux-ci ; de préférence, les charges et/ou diluants sont de la cellulose microcristalline et du phosphate dicalcique dihydraté.

13. Composition pharmaceutique de dichlorhydrate de pramipexole monohydraté selon la revendication 10, dans laquelle le liant et/ou l'adhésif est sélectionné parmi le groupe comprenant le polyvinylpyrrolidone, la gélatine, les sucres, le glucose, les gommes naturelles, les celluloses synthétiques, le polyméthycrylate, les dérivés de la cellulose tels que l'hydroxypropylméthylcellulose, l'hydroxypropylcellulose, la carboxyméthylcellulose, la méthylcellulose et équivalents et mélanges de ceux-ci ; de préférence, le liant et/ou l'adhésif est du polyvinylpyrrolidone.

14. Composition pharmaceutique de dichlorhydrate de pramipexole monohydraté selon la revendication 10, dans laquelle le glissant est sélectionné parmi le groupe comprenant le dioxyde de silicium colloïdal, le talc, le silicate d'aluminium et équivalents et mélanges de ceux-ci ; de préférence, le glissant est du dioxyde de silicium colloïdal.

15. Composition pharmaceutique de dichlorhydrate de pramipexole monohydraté selon la revendication 10, dans laquelle le lubrifiant est sélectionné parmi le groupe comprenant le stéarate de magnésium, le stéarylfumarate de sodium, le polyéthylèneglycol, l'acide stéarique, les stéarates métalliques, le talc, l'acide borique, le benzoate et l'acétate de chlorure de sodium, le lauryl sulfate de sodium ou de magnésium et équivalents et mélanges de ceux-ci ; de préférence, le lubrifiant est du stéarate de magnésium.

16. Composition pharmaceutique de dichlorhydrate de pramipexole monohydraté selon les revendications 1 à 15, comprenant :
a. 0,01 - 5,0 % de dichlorhydrate de pramipexole monohydraté
b. 5,0 - 90 % de phosphate dicalcique dihydraté
c. 0,1 - 10 % de glycolate d'amidon sodique
d. 5,0 - 90 % de cellulose microcristalline
e. 0,1 - 25 % de polyvinylpyrrolidone
f. 0,01 - 2,0 % de dioxyde de silicium colloïdal
g. 0,1 - 5,0 % de stéarate de magnésium

17. Composition pharmaceutique de dichlorhydrate de pramipexole monohydraté selon l'une quelconque des revendications précédentes, dans laquelle la composition pharmaceutique est sous la forme de comprimés, gélules, poudres, sachets ; de préférence, la forme de dosage final est sous forme de comprimé.

18. Composition pharmaceutique de dichlorhydrate de pramipexole monohydraté selon l'une quelconque des revendications précédentes, dans laquelle la forme de dosage final présente une uniformité de masse inférieure à 2,0% CV (Coefficient de variation).

19. Composition pharmaceutique de dichlorhydrate de pramipexole monohydraté selon la revendication 18, dans laquelle la forme de dosage final présente une uniformité de masse inférieure à 1,0 % CV (Coefficient de variation).

20. Composition pharmaceutique de dichlorhydrate de pramipexole monohydraté selon l'une quelconque des revendications précédentes, dans laquelle la composition pharmaceutique a une longue durée de conservation de 24 mois ou plus à température ambiante, dans son emballage d'origine.

21. Procédé pour préparer la composition pharmaceutique de dichlorhydrate de pramipexole monohydraté selon l'une quelconque des revendications précédentes, comprenant les étapes suivantes ;
a. dissoudre du dichlorhydrate de pramipexole monohydraté et du polyvinylpyrrolidone dans de l'eau et/ou de l'alcool pour former une solution aqueuse de dichlorhydrate de pramipexole monohydraté,
b. pendant que la solution est en cours de mélange, ajouter et mélanger du phosphate dicalcique dihydraté, de la cellulose microcristalline et une demi-partie de glycolate d'amidon sodique dans un granulateur à haut cisaillement pour former des granules,
c. tamiser et sécher les granules humides et moudre les granules séchés,
d. ajouter du dioxyde de silicium colloïdal et le reste du glycolate d'amidon sodique et les mélanger,
e. ajouter du stéarate de magnésium à ce mélange et mélanger jusqu'à obtention d'un mélange sec homogène,
f. comprimer le mélange composé pour former des comprimés ou remplir des gélules avec le mélange sec.

22. Procédé pour préparer la composition pharmaceutique de dichlorhydrate de pramipexole monohydraté selon l'une quelconque des revendications 1 à 20, comprenant les étapes suivantes :
a. dissoudre du dichlorhydrate de pramipexole monohydraté et du polyvinylpyrrolidone dans de l'eau et/ou de l'alcool pour former une solution aqueuse de dichlorhydrate de pramipexole monohydraté,
b. mélanger du phosphate dicalcique dihydraté, de la cellulose microcristalline et une demi-partie de glycolate d'amidon sodique dans un granulateur à haut cisaillement,
c. pulvériser la solution de dichlorhydrate de pramipexole monohydraté sur le mélange composé sec de phosphate dicalcique dihydraté, de glycolate d'amidon sodique et de cellulose microcristalline,
d. tamiser et sécher les granules humides et moudre les granules séchés,
e. ajouter du dioxyde de silicium colloïdal et le reste du glycolate d'amidon sodique et les mélanger,
f. ajouter du stéarate de magnésium à ce mélange et mélanger jusqu'à obtention d'un mélange sec homogène,
g. comprimer le mélange composé pour former des comprimés ou remplir des gélules avec le mélange sec.

23. Procédé selon l'une quelconque des revendications précédentes, dans lequel le tamis a une dimension de 0,50 mm, de préférence, 0,25 mm.

24. Utilisation de la composition pharmaceutique de dichlorhydrate de pramipexole monohydraté selon l'une quelconque des revendications précédentes pour préparer une composition pharmaceutique pour le traitement du syndrome parkinsonien ou de la maladie de Parkinson et des complications ou troubles associés à ceux-ci, de la schizophrénie et du syndrome des jambes sans repos.
